# EUROPEAN PATENT SPECIFICATION

(11) **EP 3 594 654 B1**
(45) Date of publication and mention of the grant of the patent: **14.05.2025**
(21) Application number: 18764273.1
(22) Date of filing: 28.02.2018
(51) Int. Cl.: G01N 1/00, C12M 1/00, G01N 15/00, G01N 15/14, B01L 3/00

(54) **CELL SAMPLE LIQUID FEED BAG, CELL SAMPLE LIQUID FEED METHOD, AND CELL SAMPLE LIQUID FEED DEVICE**
ZELLPROBENFLÜSSIGKEITSZUFUHRBEUTEL, ZELLPROBENFLÜSSIGKEITSZUFUHRVERFAHREN UND ZELLPROBENFLÜSSIGKEITSZUFUHRVORRICHTUNG
POCHE D'ALIMENTATION EN LIQUIDE D'ÉCHANTILLON CELLULAIRE, PROCÉDÉ D'ALIMENTATION EN LIQUIDE D'ÉCHANTILLON CELLULAIRE ET DISPOSITIF D'ALIMENTATION EN LIQUIDE D'ÉCHANTILLON CELLULAIRE

(30) Priority: 08.03.2017 JP 2017043714
(43) Date of publication of application: 15.01.2020
(73) Proprietor: Sony Group Corporation, Tokyo 108-0075 (JP)
(72) Inventor: TASHIRO Shinji, Tokyo 108-0075 (JP); HASHIMOTO Gakuji, Tokyo 108-0075 (JP); TAKAHASHI Kazuya, Tokyo 108-0075 (JP); KATSUMOTO Yoichi, Tokyo 108-0075 (JP); SAKAMOTO Naohisa, Tokyo 108-0075 (JP)
(74) Representative: D Young & Co LLP
(86) International application number: PCT/JP2018/007615
(87) International publication number: WO 2018/163943

(56) References cited:
- EP-B1- 1 633 465
- JP-A- 2000 226 513
- JP-A- 2008 216 218
- JP-A- 2010 008 384
- JP-A- 2010 230 629
- JP-A- 2011 137 693
- JP-A- 2014 210 397
- JP-A- H07 260 761
- US-A1- 2011 003 325
- US-A1- 2014 011 270
- US-A1- 2016 187 364

## Description

### TECHNICAL FIELD

The present technology relates to a cell sample liquid feeding bag, a cell sample liquid feeding method, and a cell sample liquid feeding device.

### BACKGROUND ART

In recent years, regenerative medicine/cell therapy is actively researched and there is a growing need for a flow cytometer as a technique to quickly evaluate a cell. The flow cytometer is an analysis technique in which microparticles to be analyzed are poured into a fluid in an aligned state, and the microparticles are analyzed and sorted by irradiating the microparticles with laser light or the like and detecting fluorescence and scattered light emitted from each of the microparticles, and the flow cytometer is used as a tool to analyze a cell in the research on the regenerative medicine/cell therapy. As a device of the above-described flow cytometer, for example, a microparticle measurement devices as disclosed in Patent Documents 1 to 4 are known.

Conventionally, it is known that in a case of feeding cell sample liquid without stirring the cell sample liquid at the time of the feeding, clogging easily occurs inside a tube because the high-concentration liquid is fed, and stability of a device to which the cell sample liquid is supplied is impaired. Additionally, particularly at the time of liquid feeding to a microparticle measurement device, it is also known that sorting processing cannot be executed in time and a cell sample is wasted because the high-concentration cell sample liquid flows in.

On the other hand, in a case of closed-type liquid feeding using a conventional bag, liquid is fed after stirring the liquid by using a stirring method such as shaking the bag or stirring the liquid with a stirrer. However, a rocking shaker or the like requires a space and cost. Additionally, a magnet stirrer can have a size more reduced than in the case of shaking by rocking, but there may be a risk that a stirrer rotor blade damages a cell sample. Furthermore, it is also possible to consider a stirring method by generating a reflux flow inside the bag, but a pump that generates the reflux flow is necessary, and therefore, upsizing of the device and cost increase are estimated. Thus, while stirring is indispensable for stable liquid feeding to the microparticle measurement device, it is desired to eliminate the stirring as much as possible in terms of device development.

Here, a general cell sample liquid feeding bag has a quadrangular shape, a liquid inlet/outlet port has a large tube diameter, and there is a problem of air mixture when the cell sample liquid inside the bag is sucked out, and also there is a problem that a dead volume is generated due to the liquid remaining at a corner of the bag. Additionally, the air mixture is a phenomenon desired to be prevented in feeding the cell sample liquid to the microparticle measurement device because the air mixture becomes a serious disturbance at the time acquiring a signal of a cell particle. Furthermore, it is necessary to reduce the dead volume as much as possible because samples are wasted.

### CITATION LIST

### PATENT DOCUMENT

Patent Document 1: Japanese Patent Application Laid-Open No. 2012-127922
Patent Document 2: Japanese Patent Application Laid-Open No. 2013-210287
Patent Document 3: Japanese Patent Application Laid-Open No. 2014-036604
Patent Document 4: Japanese Patent Application Laid-Open No. 2014-202573

US 2016/187364 A1 discloses a liquid suction tool, liquid supply unit and an automated analyzer that can reduce an amount of the liquid remaining in the suction portion while maintaining the strength of the suction portion. The liquid suction tool has a rod-like suction portion and a connecting member. The suction portion is inserted into the storage bag. A groove portion is formed on a side surface of the suction portion to pass the liquid.

US 2014/011270 A1 discloses an inflatable bioreactor bag for cell cultivation, comprising a top and a bottom sheet of flexible material, joined together to form two end edges and two side edges, wherein one baffle or a plurality of baffles extend from the bottom sheet in a region where the shortest distance to any one of the two end edges is higher than about one fourth of the shortest distance between the two end edges.

EP 1633465 A2 discloses a mixing bag having an interior mixing chamber having a volume sufficient to receive a predetermined weight of substantially dry material and a selected volume of process compatible solution to form a solution having the material in suspension with a selected concentration. The mixing bag includes at least two sealable openings. One opening is configured for at least one of enabling transporting the substantially dry material into the interior mixing chamber, enabling agitation of the material and process compatible solution in the interior mixing chamber to form the solution and enabling withdrawal of the solution at the selected concentration. The other opening is configured for at least one of injection of a process compatible solution into the interior mixing chamber to form the solution, enabling agitation of the material and process compatible solution in the interior mixing chamber and enabling withdrawal of the solution.

JP 2000-226513 A aims to obtain an antithrombotic polymer composition by mixing a polyamide with a specific polyalkyl ether / polyaryl ether sulfone-based copolymer. This composition comprises (A) 1-50 pts.wt. of an polyalkyl ether / polyaryl ether sulfone copolymer which is composed of repeating units of (i) the formula -Ar¹-X-Ar²-O-, (ii) the formula -Ar³-Y-Ar⁴- and (iii) the formula (-RO-)ₖ, [X is SO2; Ar¹ to Ar⁴ are each a (nucleus-substituted) 6-30C bifunctional aromatic hydrocarbon or the like; Y is a (substituted) 1-13C bifunctional aromatic hydrocarbon or the like; R is a 2-3C alkylene; and k is a number of repeating unit to make the molecular weight of the polyoxyalkylene structure of (RO)ₖ have 2,000-20,000] and has the content of the repeating unit of the formula iii to the total of the repeating units of the formula (i) to the formula (iii) of 10-90 wt.% by weight ratio and >=0.5 reduced viscosity measured in a mixed solvent of phenol/1,1,2,2-tetrachloroethane of 6/4 (weight ratio) in 1.2 g/dl concentration at 35 °C and (B) 99-50 pts.wt. of a polyamide.

JP 2014-210397 A aims to provide a method for stably introducing a high-quality hydrophilic layer capable of sufficiently inhibiting nonspecific adsorption of a bio-related substance such as protein into a plastic surface of a substrate, and provides a production method of a substrate having a hydrophilic layer on a surface thereof, which includes: a substrate preparation step of preparing a substrate having a plastic surface; a plasma treatment step 1 of exposing the plastic surface of the substrate to plasma containing rare gas; a plasma treatment step 2 of exposing the plastic surface of the substrate to plasma containing fluorocarbon gas; a primer layer formation step of forming a primer layer containing polysiloxane by polymerizing a silanol compound having an organic group that contains a carbon atom directly bonded to a silicon atom and has a functional group, on the surface of the substrate exposed to plasma; and a hydrophilic layer formation step of linking the primer layer with a hydrophilic polymer via a covalent bond by the reaction of the functional group on a side chain of the polysiloxane with a functional group of the hydrophilic polymer.

### SUMMARY OF THE INVENTION

### PROBLEMS TO BE SOLVED BY THE INVENTION

Considering the above-described situation, it is desired to develop a cell sample liquid feeding bag having a new shape, a feeding method thereof, and a feeding device thereof which enable stable liquid feeding.

Therefore, the present technology is mainly directed to providing a technology that enables stable liquid feeding.

### SOLUTIONS TO PROBLEMS

The problems are solved by the subject matter of the independent claims.

The present technology first provides a cell sample liquid feeding bag including at least: an outflow port from which cell sample liquid flows out; a bottom portion including a reservoir capable of storing cells, and at least partly including a slope; and a first inner tube extending from the outflow port toward the reservoir up to a position not contacting the reservoir, in which the cell sample liquid is fed from a reservoir side toward an outflow port side of the first inner tube.

In the cell sample liquid feeding bag according to the present technology, a cross-sectional shape of the bottom portion has a substantially V-shape.

Additionally, the cell sample liquid feeding bag according to the present technology may further include an outer tube extending from the outflow port toward outside of the bag, and the outer tube may include at least two or more branched paths.

Furthermore, the cell sample liquid feeding bag according to the present technology may further include at least one or more ports besides the outflow port. In this case, the ports may be provided at a bag wall surface. Additionally, in this case, one of the ports may further include a second inner tube extending toward the inside of the bag, and the second inner tube may be used for cell sample liquid injection. Furthermore, in this case, an inner diameter of the second inner tube may be larger than an inner diameter of the first inner tube.

Additionally, the cell sample liquid feeding bag according to the present technology may have at least a part of a bag inner wall applied with a coating that inhibits nonspecific adsorption of the sample. In this case, the coating may include one kind selected from a group consisting of low molecular protein, silicon, and a water-soluble polymer. Additionally, in this case, the low molecular protein may include albumin, and the water-soluble polymer may include at least one or more kinds selected from a group consisting of casein, gelatin, dextran, polyacrylamide, polyvinyl alcohol, polyvinylpyrrolidone, and polyethylene glycol.

Furthermore, the cell sample liquid feeding bag according to the present technology may further include a support portion that supports an upright posture of the bag.

Additionally, the present technology also provides a cell sample liquid feeding method using a cell sample liquid feeding bag according to claim 1 and the method at least includes: an ejecting step of ejecting liquid toward the reservoir before starting cell sample liquid feeding.

In the cell sample liquid feeding method according to the present technology, the cell sample liquid feeding bag may further include an outer tube extending from the outflow port toward the outside of the bag, and the ejection may be executed by reverse rotation of an outer tube pump.

Furthermore, the present technology also provides a cell sample liquid feeding device including at least a cell sample liquid feeding bag including at least a cell sample liquid feeding bag according to claim 1.

The cell sample liquid feeding device according to the present technology may further include an ejection mechanism capable of ejecting liquid toward the reservoir before feeding the cell sample liquid. In this case, the cell sample liquid feeding bag further includes an outer tube extending from the outflow port toward the outside of the bag, and the ejection mechanism is run by reverse rotation of an outer tube pump.

Furthermore, the present technology also provides a microparticle measurement device including at least the cell sample liquid feeding device according to the present technology.

In the present technology, the "microparticle" broadly includes, for example: biologically relevant microparticles such as cells, microbes, and liposomes; synthetic particles such as a latex particle, a gel particle, and a particle for an industrial use; and the like.

Additionally, the biologically relevant microparticles include a chromosome, a liposome, a mitochondrion, an organelle (cell organ), and the like constituting various kinds of cells. The cells include animal cells (such as hematopoietic cell) and plant cells. The microbes include: bacteria such as coli bacilli; viruses such as tobacco mosaic viruses; fungi such as yeast; and the like. Additionally, the biologically relevant microparticles also include biologically relevant polymers such as nucleic acids, proteins, and a complex thereof. Additionally, the particle for the industrial use may include, for example, an organic or inorganic polymer material, a metal, or the like. The organic polymer materials include polystyrene, styrenedivinylbenzene, polymethyl methacrylate, and the like. The inorganic polymer materials include glass, silica, a magnetic material, and the like. The metal includes gold colloid, aluminum, and the like. Generally, these microparticles each have a spherical shape, but in the present technology, the shape may be non-spherical, and a size, mass, and the like thereof are not particularly limited.

### EFFECTS OF THE INVENTION

According to the present technology, the liquid can be stably fed. Note that the effect recited herein is not necessarily limited and may be any one of those recited in the present disclosure.

### BRIEF DESCRIPTION OF DRAWINGS

Fig. 1 is a schematic view illustrating a first embodiment of a cell sample liquid feeding bag 1 according to the present technology.
Fig. 2 is a schematic view illustrating a second embodiment of a cell sample liquid feeding bag 1 according to the present technology.
Fig. 3 is a schematic view illustrating a third embodiment of a cell sample liquid feeding bag 1 according to the present technology.
Fig. 4 is a schematic view illustrating a fourth embodiment of a cell sample liquid feeding bag 1 according to the present technology.
Fig. 5 is a schematic view illustrating a fifth embodiment of a cell sample liquid feeding bag 1 according to the present technology.
Fig. 6 is a schematic view illustrating a sixth embodiment of a cell sample liquid feeding bag 1 according to the present technology.
Fig. 7 is a schematic view illustrating a seventh embodiment of a cell sample liquid feeding bag 1 according to the present technology.
Fig. 8 is a schematic view illustrating an eighth embodiment of a cell sample liquid feeding bag 1 according to the present technology.
Fig. 9 is a front view of a ninth embodiment of a cell sample liquid feeding bag 1 according to the present technology. Note that a rear view is the same as the front view.
Fig. 10 is a right side view of the ninth embodiment of the cell sample liquid feeding bag 1 according to the present technology. Note that a left side view is the same as the right side view.
Fig. 11 is a plan view of the ninth embodiment of the cell sample liquid feeding bag 1 according to the present technology.
Fig. 12 is a bottom view of the ninth embodiment of the cell sample liquid feeding bag 1 according to the present technology.
Fig. 13 is a cross-sectional view taken along a line A-A of the ninth embodiment of the cell sample liquid feeding bag 1 according to the present technology.
Fig. 14 is a partial enlarged view of a portion between B-B of the ninth embodiment of the cell sample liquid feeding bag 1 according to the present technology.
Fig. 15 is a perspective view of the ninth embodiment of the cell sample liquid feeding bag 1 according to the present technology.
A to E of Fig. 16 are modified examples of the ninth embodiment of the cell sample liquid feeding bag 1 according to the present technology.
Fig. 17 is a front view of a tenth embodiment of a cell sample liquid feeding bag 1 according to the present technology. Note that a rear view is the same as the front view.
Fig. 18 is a right side view of the tenth embodiment of the cell sample liquid feeding bag 1 according to the present technology. Note that a left side view is the same as the right side view.
Fig. 19 is a plan view of the tenth embodiment of the cell sample liquid feeding bag 1 according to the present technology.
Fig. 20 is a bottom view of the tenth embodiment of the cell sample liquid feeding bag 1 according to the present technology.
Fig. 21 is a perspective view of the tenth embodiment of the cell sample liquid feeding bag 1 according to the present technology.
A to E of Fig. 22 are modified examples of the tenth embodiment of the cell sample liquid feeding bag 1 according to the present technology.
Fig. 23 is a referential view illustrating an exemplary flow form of the cell sample liquid feeding bag 1 according to the present technology.
Fig. 24 is a graph prepared as a drawing and illustrating a result of studying achievement of a uniform cell sample concentration by an ejection process.
Fig. 25 is a schematic conceptual view schematically illustrating an exemplary embodiment of the ejection process.
Fig. 26 is a schematic conceptual diagram schematically illustrating an exemplary embodiment of a microparticle measurement device 100 according to the present technology.
A and B of Fig. 27 are schematic views illustrating an exemplary configuration of a microparticle measurement chip M that can be used in the microparticle measurement device 100 of Fig. 26.
A to C of Fig. 28 are schematic views illustrating an exemplary configuration of an orifice M1 of the microparticle measurement chip M that can be used in the microparticle measurement device 100 of Fig. 26.

### MODE FOR CARRYING OUT THE INVENTION

Preferred embodiments to carry out the present technology will be described below with reference to the drawings. Note that the embodiments described below illustrate examples of the representative embodiments of the present technology and the scope of the present technology should not be interpreted to be limited by these embodiments. Note that the description will be provided in the following order.
1. Cell Sample Liquid Feeding Bag 1
2. Cell Sample Liquid Feeding Method
   (1) Ejection Process
   (2) Other Processes
3. Cell Sample Liquid Feeding Device 10
4. Microparticle Measurement Device 100
   (1) Flow Path P
      (1-1) Microparticle Measurement Chip M
   (2) Fluid Control Unit 101
   (3) Light Emission Unit 102
   (4) Light Detection Unit 103
   (5) Analysis Unit 104
   (6) Sorting Unit 105
   (7) Memory Unit 106
   (8) Display Unit 107
   (9) Input Unit 108
   (10) Control Unit 109
   (11) Others

### 1. Cell Sample Liquid Feeding Bag 1

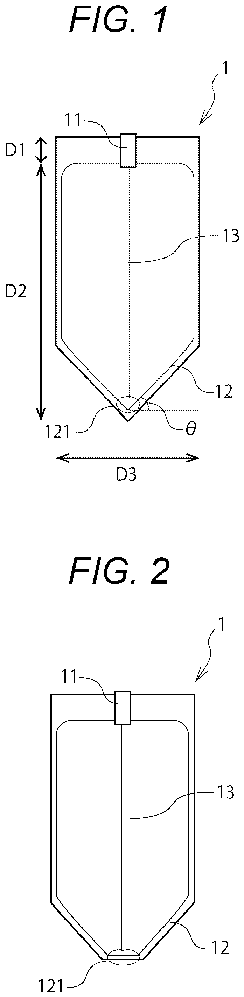

Fig. 1 is a schematic view illustrating a first embodiment of a cell sample liquid feeding bag 1 according to the present technology. The cell sample liquid feeding bag 1 according to the present technology includes at least: an outflow port 11 from which cell sample liquid flows out; a reservoir 121 capable of storing cells; a bottom portion 12 at least partly including a slope; and a first inner tube 13 extending from the outflow port 11 toward the reservoir 121 up to a position not contacting the reservoir 121. Additionally, the cell sample liquid is fed from the reservoir 121 side toward the outflow port 11 side of the first inner tube 13.

Conventionally, shake stirring by rocking, or the like is executed as a method of stirring cells inside a closed-type bag, however; since the cell sample liquid feeding bag 1 according to the present technology is used, a cell sample that has settled in the reservoir 121 is whirled up and the cell sample can be kept in a stirred state constantly having a uniform concentration without using a stirrer, and as a result, the liquid can be stably fed. For this reason, downsizing of the device and reduction of a manufacturing cost can be achieved. Furthermore, the cell sample inside the bag can be fed while reducing a dead volume.

Additionally, since a conventional cell sample liquid feeding bag adopts a form in which a liquid feeding port is located at a lowermost end in a hung state and liquid is sucked from below, cells are made to settle at the time of liquid feeding without stirring, and furthermore, a sample flow is very slow such as about 100 µL/min, and a thin feeding tube (for example, φ 0.1 - 0.25 mm or the like) is also used, and therefore, there is a high possibility that the tube is clogged in a direction sucking out the liquid downward from the lowermost end before starting the liquid feeding. On the other hand, in the cell sample liquid feeding bag 1 according to the present technology, the cell sample liquid is fed from the reservoir 121 side toward the outflow port 11 side of the first inner tube 13, and therefore, a tube is hung from an uppermost end to a lowermost end and a sucking portion has a shape sucking the liquid with the tube from a lower side to an upper side, and the possibility of clog inside the tube can be reduced before start the feeding.

Furthermore, when the cell sample liquid feeding bag 1 according to the present technology is used, it is sufficient that the bag is set upright. Therefore, a degree of freedom relative to a method of fixing the bag to a device is more increased than in the conventional cell sample liquid feeding bag. Additionally, as a method of setting the bag upright, for example, it is possible to exemplify; a method of hanging the bag at a hook or the like; putting the bag in a container or the like and fixing the bag inside the container; or the like. Furthermore, besides the above, there also is a method of providing a support portion 17 described later.

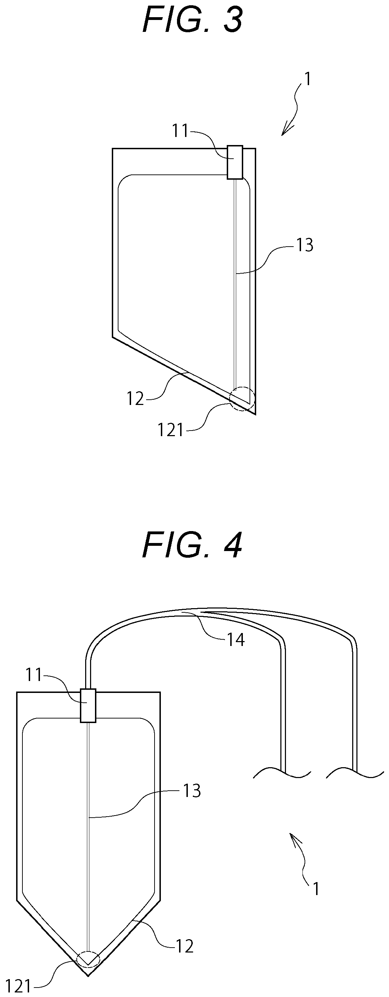

A shape of the bottom portion 12 is not particularly limited as far as the shape includes the reservoir 121 capable of storing the cells, and at least partly includes a slope, and a shape of a second embodiment as illustrated in Fig. 2 or a shape of a third embodiment illustrated in Fig. 3 or the like may also be adopted. Since the bottom portion 12 has the above-described shape, precipitated cells can be collected in the reservoir 121, and therefore, the cells that are settling can be fed as much as possible without waste.

A cross-sectional shape of the bottom portion 12 is not also particularly limited but preferably has a substantially V-shape as illustrated in the first embodiment of Fig. 1. With this cross-sectional shape, the precipitated cells can be more efficiently collected in the reservoir 121. Additionally, in this case, an angle (inclination angle) θ (see Fig. 1) between a horizontal plane and the cross section of the bottom portion 121 is also not particularly limited but is preferably 50° to 80°, more preferably 55° to 75°, and particularly preferably 60° to 70°.

Lengths of D1, D2, and D3 illustrated in Fig. 1 are also not particularly limited, and for example, D1 can be 20 mm, D2 can be 100 mm, and D3 can be 35 mm. A reason why D1 is set to 20 mm is to secure a margin for making a hole because there may be a case where it becomes necessary to make a hole in an upper portion of the bag when the bag is used in a hung state.

Additionally, in the present technology, a distance between the reservoir 121 and the first inner tube 13 is not particularly limited and may be, for example, about 5 mm.

Furthermore, in the present technology, an outer diameter, an inner diameter, a material, and the like of the first inner tube 13 are also not particularly limited, and for example, a PEEK tube having an outer diameter of 1/16 inches, an inner diameter of 0.5 mm or 1 mm, or the like can be used as the first inner tube 13.

In the present technology, as illustrated in a fourth embodiment of Fig. 4, an outer tube 14 extending from the outflow port 11 toward the outside of the bag is further provided, and the outer tube 14 can include at least two or more branched paths. With the branched paths, it is possible to separately provide a tube used at the time of injecting the cell sample into the bag and a tube used at the time of sucking out the cell sample, and the tube used at the time of injecting the cells can be aseptically cut and closed after the injection, and therefore, a risk of contamination can be reduced and convenience of a user can be improved.

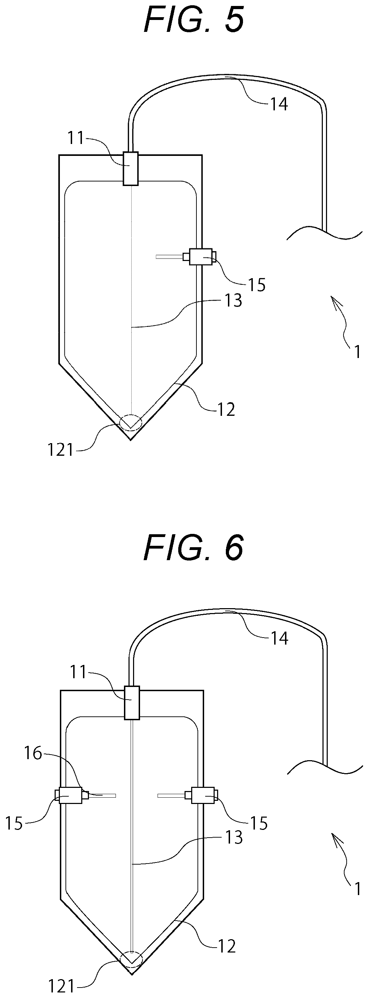

Note that, in the present technology, a shape of the outer tube 14 is not limited to a shape illustrated in the fourth embodiment of Fig. 4, and may not necessarily include the branched paths as illustrated in a fifth embodiment of Fig. 5.

Additionally, in the present technology, an inner diameter, a length, a material, and the like of the outer tube 14 are not particularly limited, and for example, a medical tube having an inner diameter of 1 mm, a length of 100 to 200 mm, and including polyethylene (PE), polyvinyl chloride (PVC), thermoplastic elastomer (TPE), or the like can be used as the outer tube 15.

Furthermore, in the present technology, as illustrated in the fifth embodiment of Fig. 5, at least one or more ports 15 can be further provided besides the outflow port 11. With this structure, the port 15 can be used, for example, as an injection port at the time of injecting the cell sample into the bag, and as for the port 15 used for the injection, a tube connected to the port 15 can be aseptically cut and closed after the injection, and therefore reduce the risk of contamination can be reduced and the convenience of a user can be improved.

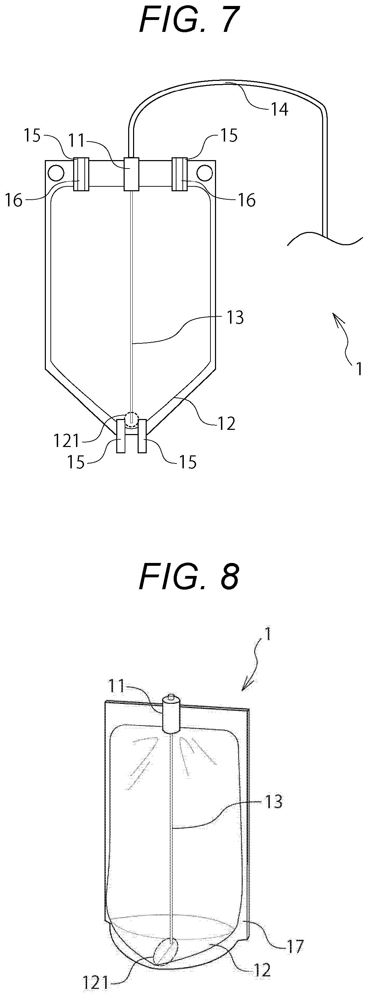

In a case where the cell sample liquid feeding bag 1 according to the present technology includes the port(s) 15 as illustrated in the fifth embodiment of Fig. 5, a sixth embodiment of Fig. 6, and a seventh embodiment of Fig. 7, it is preferable that the port(s) 15 be provided at a bag wall surface.

Additionally, in a case where the ports 15 are provided at the bag wall surface, one of the ports 15 further includes a second inner tube 16 extending toward the inside of the bag as illustrated in the sixth embodiment of Fig. 6, and the second inner tube 16 can also be used for cell sample liquid injection. This improves the convenience of a user.

Furthermore, in this case, in the present technology, an inner diameter of the second inner tube 16 is preferably larger than the inner diameter of the first inner tube 13. With this large inner diameter, pressure drop is reduced and it becomes easy to feed the cell sample at the time of injecting the cell sample faster than at the time of sucking out the cell sample.

Additionally, as illustrated in the sixth embodiment of Fig. 6, in the case where the cell sample liquid feeding bag 1 according to the present technology includes the two or more ports 15, one of the ports 15 may be used for the cell sample liquid injection, and the other port(s) 15 may be used to inject liquid such as reagent into the bag or inject a dyeing solution, a transgenic virus solution, or the like. Furthermore, as for any of the ports 15, it is possible to aseptically cut and close the tube connected to each of the ports 15 after the injection, and therefore, the risk of contamination can be reduced or the convenience of a user can be improved.

As illustrated in the seventh embodiment of Fig. 7, in the case where the cell sample liquid feeding bag 1 according to the present technology includes the two or more ports 15, the number and installation positions thereof are also not particularly limited as far as the ports are provided at the bag wall surface. In the present technology, as illustrated in the seventh embodiment, the plurality of ports 15 can be provided on a left side and a right side of the outflow port 11 and in the vicinity of a tip of the bottom surface 12. Also, as illustrated in the present embodiment, each second inner tube 16 may extend through the inside of each of the ports 15 toward the inside of the bag.

In the present technology, at least a part of the bag inner wall can be applied with a coating that inhibits nonspecific adsorption of the sample. It is known that cells generally cause nonspecific adsorption, and therefore, it is assumed that there are cells that nonspecifically adsorb also onto the bag inner wall and fail to fall down to the reservoir 121. Accordingly, by applying the coating in order to reduce the nonspecific adsorption, the cells can be collected, made to settle, it is possible to more efficiently collect the cells and make the cells settle and be accumulated in the reservoir 121. With this effect, the cell sample having a stable concentration can be continuously supplied in a case of executing a cell sample liquid feeding method described later.

In the present technology, the coating is not particularly limited, but it is preferable to use one kind selected from a group consisting of low molecular protein, silicon, and a water-soluble polymer. Additionally, the low molecular protein preferably includes albumin, and the water-soluble polymer includes at least one or more kinds selected from a group consisting of casein, gelatin, dextran, polyacrylamide, polyvinyl alcohol, polyvinylpyrrolidone, and polyethylene glycol.

Also, in the present technology, at least a part of the bag inner wall can also be applied with a coating that promotes specific adsorption of the sample. With this coating, it is possible to trap unnecessary cells and improve purity of necessary cells in a collected solution.

The cell sample liquid feeding bag 1 according to the present technology may further include the support portion 17 that supports an upright posture of the bag. As described above, the cell sample liquid feeding bag 1 according to the present technology is required to be used in the upright state, and as one of methods thereof, it may be conceivable to devise a shape of the bag such that the bag is made to stand on its own by the support portion 17.

The shape of the support portion 17 is not particularly limited, but as illustrated in an eighth embodiment of Fig. 8, it is preferable that the bottom portion 12 of the bag be provided with an outer edge.

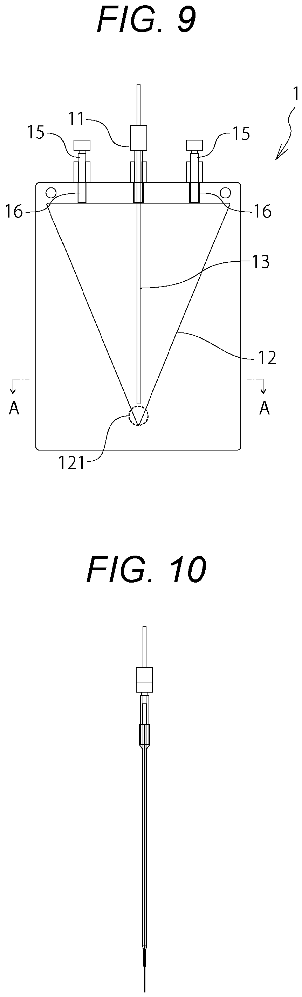

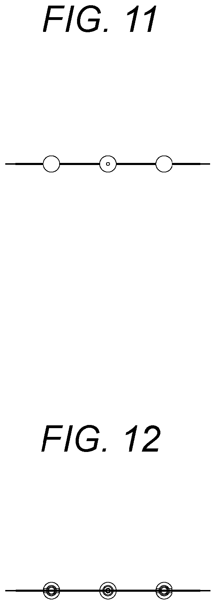

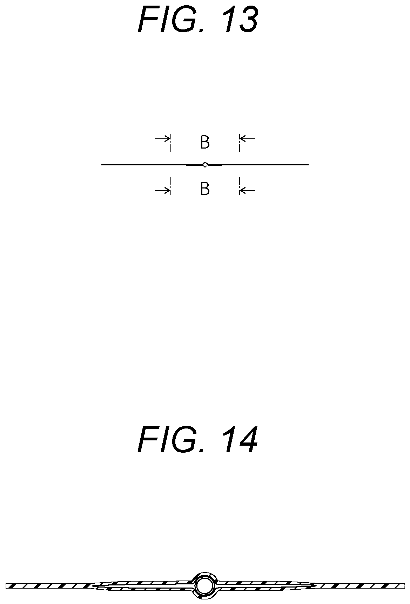

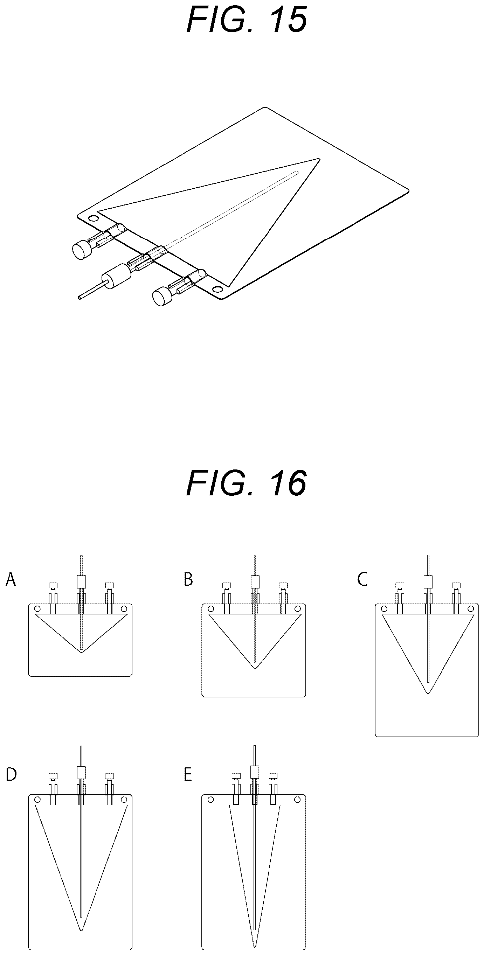

Figs. 9 to 15 illustrate a ninth embodiment of a cell sample liquid feeding bag 1 according to the present technology. Additionally, A to E of Fig. 16 are modified examples of the ninth embodiment of the cell sample liquid feeding bag 1 according to the present technology. The cell sample liquid feeding bag 1 according to the present technology can have a structure in which two transparent flexible films are partly bonded together as illustrated in the ninth embodiment.

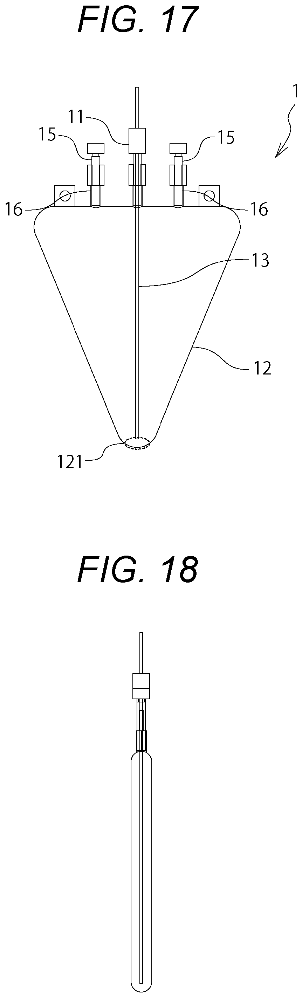

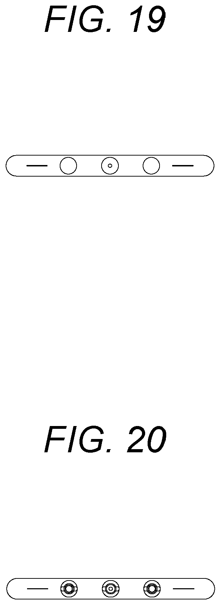

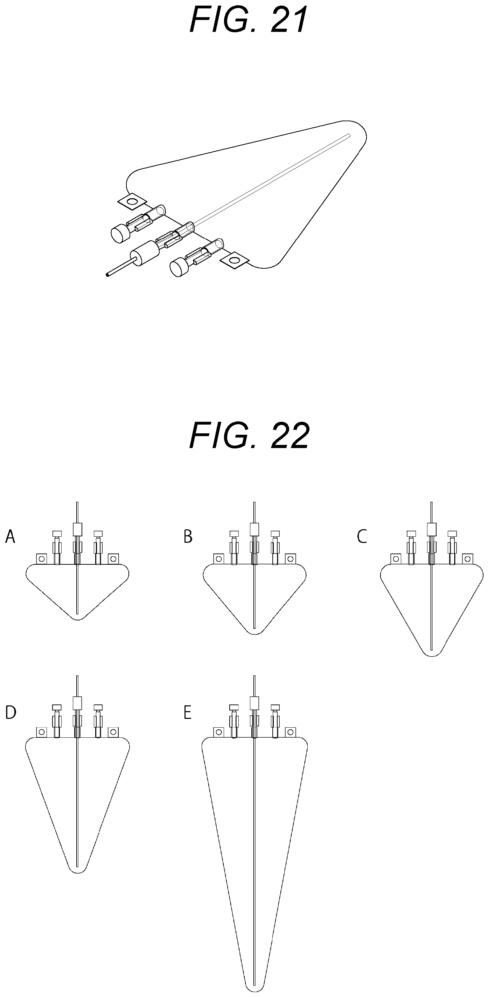

Figs. 17 to 21 illustrate a tenth embodiment of a cell sample liquid feeding bag 1 according to the present technology. Additionally, A to E of Fig. 22 are modified examples of the tenth embodiment of the cell sample liquid feeding bag 1 according to the present technology. The cell sample liquid feeding bag 1 according to the present technology can also formed by using one transparent flexible film as illustrated in the tenth embodiment.

The cell sample liquid feeding bag 1 according to the present technology may have a hole used to hang the cell sample liquid feeding bag 1 at a hook or the like as illustrated in the ninth embodiment, the tenth embodiment, and the like described above.

A product name when the cell sample liquid feeding bag 1 according to the present technology is distributed is not particularly limited, and can be named as a medical bag, a medical soft bag, a blood bag, an infusion bag, a culture bag, a medical container, a medication container, an infusion containers, and the like, for example.

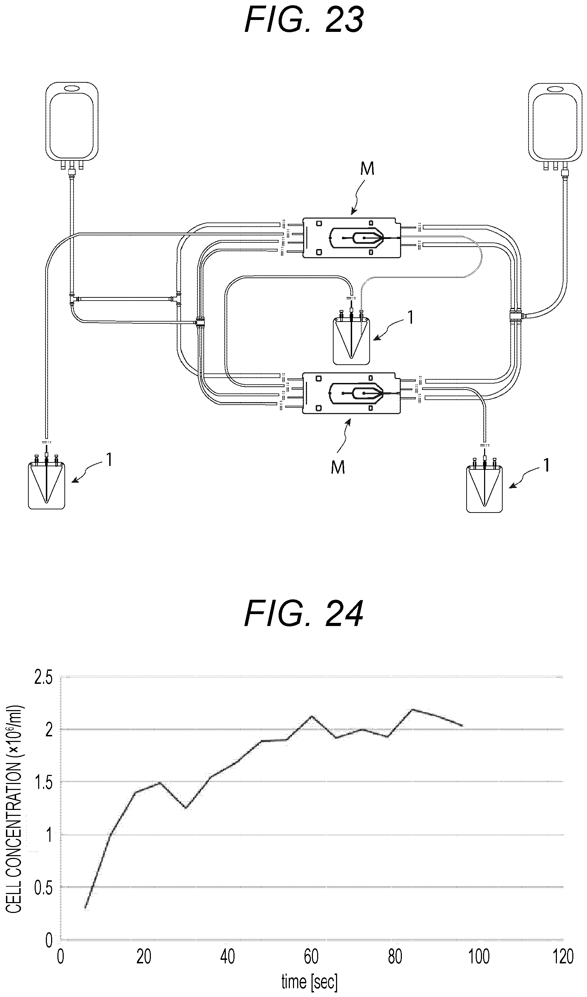

Fig. 23 is a referential view illustrating an exemplary flow form of the cell sample liquid feeding bag 1 according to the present technology. As illustrated in Fig. 23, the cell sample liquid feeding bag 1 according to the present technology may be distributed as a part of a product, such as a cartridge, a unit, a device, a kit, and an instrument for a closed-type cell sorter. Note that the number of the cell sample liquid feeding bags 1 according to the present technology are three in Fig. 23, but the number of this products is not particularly limited. Additionally, as illustrated in Fig. 23, a waste solution bag, a sheath liquid bag, a microchip, or the like may be individually connected to this product, and the number thereof is also not particularly limited.

### 2. Cell Sample Liquid Feeding Method

In the cell sample liquid feeding method according to the present technology, at least an ejection process using the above-described cell sample liquid feeding bag 1 is executed. Note that the description thereof is omitted here because the cell sample liquid feeding bag 1 is similar to the one described above.

### (1) Ejection Process

The ejection process is a process to eject liquid toward the reservoir 121 before starting the cell sample liquid feeding. With the execution of the ejection process, the cell sample liquid concentration can be made uniform by whirling up the cell sample that has settled in the reservoir 121 by leaving the cell sample liquid feeding bag 1 stationary, and stable liquid feeding can be performed. Fig. 24 is a graph prepared as a drawing and illustrating a result of studying achievement of a uniform cell sample concentration by the ejection process. In Fig. 24, a vertical axis represents the cell (sample) concentration (× 10⁶/mL), and a horizontal axis represents time (sec).

As illustrated in Fig. 24, it can be confirmed that feeding of a low-concentration cell sample starts with stirring executed by ejecting the liquid at the beginning of the ejection process, and then the concentration gradually becomes constant. Note that an amount of the liquid ejected in the ejection process is not particularly limited, and for example, a maximum amount can be set, as a guide, to an amount of about 1/4 of a volume of the cell sample liquid feeding bag 1.

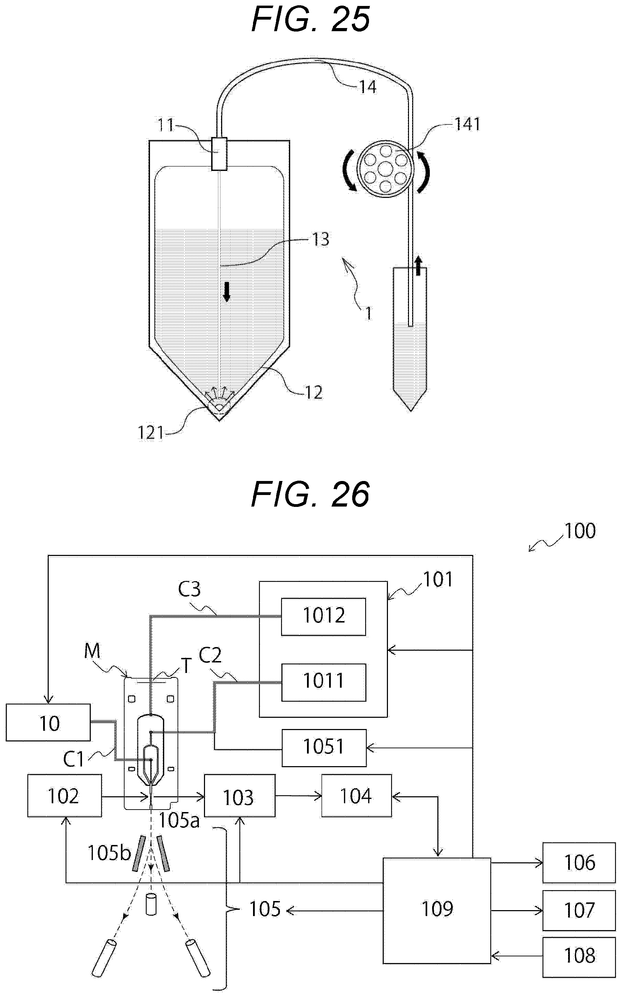

Fig. 25 is a schematic conceptual view schematically illustrating an exemplary embodiment of the ejection process. In the cell sample liquid feeding method according to the present technology, as illustrated in Fig. 25, the cell sample liquid feeding bag 1 further includes the outer tube 14 extending from the outflow port 11 toward the outside of the bag, and the ejection can be executed by reverse rotation of an outer tube pump 141. In the embodiment illustrated in Fig. 25, the liquid to be ejected is prepared at a tip of the outer tube, and the outer tube pump 141 is reversely rotated before starting the cell sample liquid feeding, thereby whirling up the cell sample that has settled in the reservoir 121. With this process, the cell sample concentration can be made uniform as described above, and it is possible to prepare start of the liquid feeding.

### (2) Other Processes

In the present technology, other processes may be executed in addition to the above-described ejection process as far as the effects of the present technology are not impaired. The other processes include, for example, methods and the like executed by a fluid control unit 101, a light emission unit 102, a light detection unit 103, an analysis unit 104, a sorting unit 105, an electric charging unit 1051, a recording unit 106, and a display unit 107, an input unit 108, a control unit 109, and the like in a microparticle measurement device 100 described later.

### 3. Cell Sample Liquid Feeding Device 10

A cell sample liquid feeding device 10 according to the present technology at least includes the above-described cell sample liquid feeding bag 1. Note that the description thereof is omitted here because the cell sample liquid feeding bag 1 is similar to the one described above.

The cell sample liquid feeding device 10 according to the present technology may further include an ejection mechanism capable of ejecting liquid toward the reservoir 121 before feeding the cell sample liquid. Since the ejection mechanism is provided, the cell sample liquid concentration can be made uniform by whirling up the cell sample that has settled in the reservoir 121 by leaving the cell sample liquid feeding bag 1 stationary, and stable liquid feeding can be performed.

Additionally, in the cell sample liquid feeding device 10 according to the present technology, as illustrated in Fig. 25 described above, the cell sample liquid feeding bag 1 further includes the outer tube 14 extending from the outflow port toward the outside of the bag, and the ejection mechanism can be run by reverse rotation 141 of the outer tube pump. With this ejection mechanism, the cell sample concentration can be made uniform as described above, and it is possible to prepare start of the liquid feeding.

The cell sample liquid feeding device 10 may have a function to feed the sample to a sample introduction unit M3 described later via a liquid feeding tube, besides the above-described functions. For example, the cell sample liquid feeding device 10 can suck/feed the sample via a nozzle from a test tube, a well plate, or the like containing the sample, or can also feed the sample by applying pressure to a housing unit that can house the test tube or the like containing the sample.

### 4. Microparticle Measurement Device 100

Fig. 26 is a schematic conceptual diagram schematically illustrating an exemplary embodiment of the microparticle measurement device 100 according to the present technology. The microparticle measurement device 100 according to the present technology includes at least the above-described cell sample liquid feeding device 10. Additionally, as necessary, a flow path P, the fluid control unit 101, the light emission unit 102, the light detection unit 103, the analysis unit 104, the sorting unit 105, the electric charging unit 1051, recording unit 106, the display unit 107, the input unit 108, the control unit 109, and the like may be provided.

In Fig. 26, a liquid feeding tube C1 capable of feeding liquid from the cell sample liquid feeding device 10, a sheath liquid feeding tube C2 capable of feeding liquid from a sheath liquid feeding unit 1011, and a liquid drain tube C3 capable of draining liquid to a liquid drain unit 1012 can be detached as necessary, and these members are disposable. Note that, in the present technology, a part or all of the liquid feeding tube C1 may be similar to the above-described outer tube 14. Furthermore, a microparticle measurement chip M described later is also disposable as necessary.

Each of the units will be described in detail below.

### (1) Flow Path P

The flow path P may be provided in advance in the microparticle measurement device 100 according to the present technology. However, a commercially-available flow path P, a chip provided with the flow path P, or the like can be installed in the microparticle measurement device 100 to execute analysis and sorting.

A form of the flow path P that can be used in the microparticle measurement device 100 according to the present technology is not particularly limited and can be freely designed. In the present technology, it is particularly preferable to use a flow path P formed inside a substrate T of a two-dimensional or three-dimensional plastic, glass, or the like as illustrated in the microparticle measurement device 100 of Fig. 26.

Additionally, a flow path width, a flow path depth, a flow path cross-sectional shape, and the like of the flow path P are also not particularly limited and can be freely designed as far as a laminar flow can be formed. For example, a micro flow path having a flow path width of 1 mm or less can also be used in the microparticle measurement device 100 according to the present technology. Particularly, a micro flow path having a flow path width of about 10 µm or more and about 1 mm or less can be preferably used in the microparticle measurement device 100 according to the present technology.

### (1-1) Microparticle Measurement Chip M

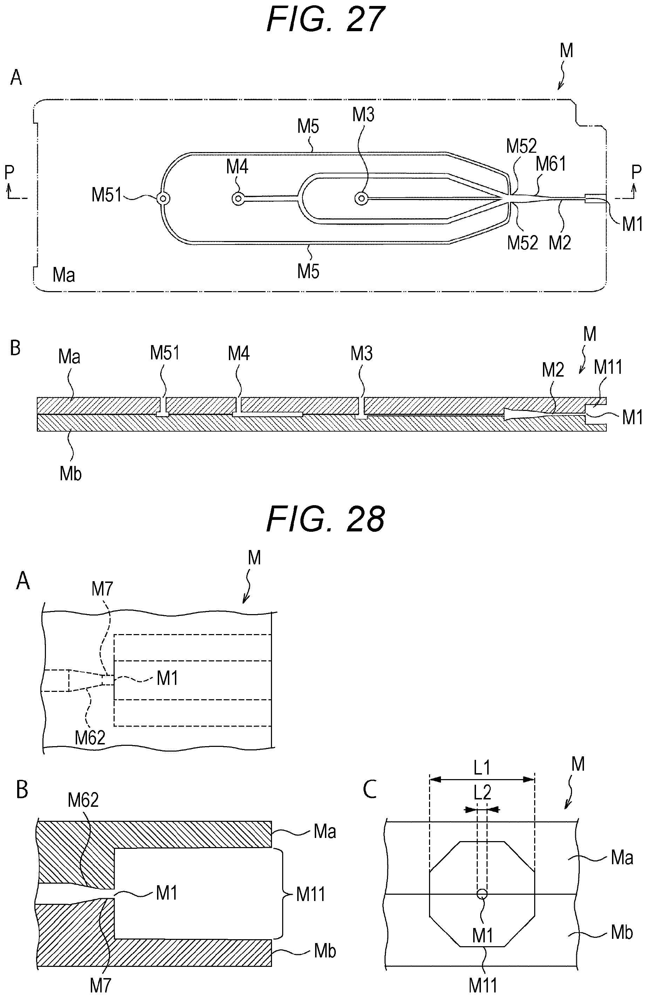

Fig. 27 is a schematic view illustrating an exemplary configuration of the microparticle measurement chip M that can be used in the microparticle measurement device 100 of Fig. 26, and Fig. 28 is a schematic view illustrating an exemplary configuration of an orifice M1 of the microparticle measurement chip M that can be used in the microparticle measurement device 100 of Fig. 26. A of Fig. 27 illustrates a schematic top view, and B of Fig. 27 illustrates a schematic cross-sectional view corresponding to a cross-section P-P of A. Also, A in Fig. 28 illustrates a top view, B of Fig. 28 is a cross-sectional view, and C of Fig. 28 is a front view. Note that B of Fig. 28 corresponds to the cross-section P-P in A of Fig. 27.

The microparticle measurement chip M is formed by bonding substrate layers Ma and Mb where a sample flow path M2 is formed. The sample flow path M2 can be formed on the substrate layers Ma and Mb by performing injection molding with a thermoplastic resin by using a metal mold. As the thermoplastic resin, it is possible to adopt plastic conventionally known as a material of a microparticle measurement chip, such as polycarbonate, polymethylmethacrylate resin (PMMA), cyclic polyolefin, polyethylene, polystyrene, polypropylene, or polydimethylsiloxane (PDMS).

Additionally, in the microparticle measurement chip M, the sample introduction unit M3 to introduce the sample containing microparticles, a sheath introduction unit M4 to introduce the sheath liquid, and the sample flow path M2 in which a sample flow is introduced and joined with the sheath liquid are formed. The sheath liquid introduced from the sheath introduction unit M4 is fed in two separate directions, and then joined with the sample liquid in a manner interposing the sample liquid between the two directions at a joined portion with the sample liquid introduced from the sample introduction unit M3. Consequently, a three-dimensional laminar flow in which the sample liquid laminar flow is positioned in a middle of sheath liquid laminar flows is formed at the joined portion.

Reference sign M5 illustrated in A of Fig. 27 represents a suction flow path in order to eliminate clogging or bubbles by temporarily reversing a flow by applying negative pressure to the inside of the sample flow path M2 in the event of the clogging or the bubbles in the sample flow path M2. The suction flow path M5 has one end formed with a suction open portion M51 connected to a negative pressure source such as a vacuum pump. Additionally, the suction flow path M5 has the other end connected to the sample flow path M2 at a communication port M52.

A laminar flow width of the three-dimensional laminar flow is narrowed at narrowed portions M61 (see Fig. 27) and M62 (see A and B of Fig. 28) each formed such that the area of a vertical cross-section relative to a liquid feeding direction becomes gradually reduced from an upstream side to a downstream side in the liquid feeding direction. After that, the three-dimensional laminar flow is drained as a fluid stream from the orifice M1 provided at one end of the flow path.

The fluid stream jetted from the orifice M1 is made into droplets by applying vibration to the orifice M by a vibration element 105a described below. The orifice M1 is opened in a direction to end surfaces of the substrate layers Ma and Mb, and a cut-away portion M11 is provided between the opened position of the orifice and the end surfaces of the substrate layers. The cut-away portion M11 is formed by cutting the substrate layers Ma and Mb between the opened position of the orifice M1 and the substrate end surfaces such that a diameter L1 of the cut-away portion M11 becomes larger than an opening diameter L2 of the orifice M1 (see C in Fig. 28). Preferably, the diameter L1 of the cut-away portion M11 is formed twice larger or more times larger than the opening diameter L2 of the orifice M1 so as not to interfere with movement of droplets discharged from the orifice M1.

### (2) Fluid Control Unit 101

The fluid control unit 101 includes the sheath liquid feeding unit 1011 to introduce the sheath liquid to the sheath liquid introduction unit M4. The sheath liquid feeding unit 1011 includes, for example: a support portion to which a sheath liquid storing unit can be attached: and a sealing portion, and the sheath liquid inside the sheath liquid storing unit is fed to the above-described sheath liquid introduction unit M4 via the sheath liquid feeding tube by pressure to the sealing portion.

The fluid control unit 101 may further include the liquid drain unit 1012. The liquid drain unit 1012 collects, for example, clogged matters, bubbles, and the like inside the sample flow path from the suction open portion via the liquid drain tube by a pump function or the like. Additionally, the liquid drain unit 1012 can also be connected to the sorting unit 105 in order to suck a droplet, aerosol, and the like not sorted at the sorting unit 105 described below.

Additionally, the fluid control unit 101 may include an installation table on which the sheath liquid feeding unit 1011 and the liquid drain unit 1012 can be installed. Furthermore, the fluid control unit 101 may be formed separately from the microparticle measurement device 100 or may be formed as a part of the microparticle measurement device 100.

### (3) Light Emission Unit 102

The light emission unit 102 emits light to a microparticle to be analyzed. A kind of light emitted from the light emission unit 102 is not particularly limited, but light having a constant light direction, a constant wavelength, and constant light intensity is preferable in order to reliably generate fluorescence and scattered light from a particle. Specifically, for example, a laser, an LED, or the like can be exemplified. In a case of using the laser, a kind thereof is not particularly limited, but it is also possible to use one kind or two or more kinds of combination of: an argon (Ar) ion laser, a helium-neon (He-Ne) laser, a dye laser, a krypton (Cr) laser, a semiconductor laser, a solid laser combining a semiconductor laser with a wavelength conversion optical element, or the like.

### (4) Light Detection Unit 103

The light detection unit 103 detects the light generated from the microparticle. The light detection unit 103 detects light components of fluorescence, forward scattered light, backscattered light, and the like generated from the microparticle in response to light emission to the microparticle from the light emission unit 102. The components of the fluorescence and necessary scattered light are important light components to obtain optical information (characteristics) of the microparticle P.

As far as light from each microparticle can be detected, a type of the light detection unit 103 is not particularly limited, and a known photodetector can be freely selected and adopted. For example, one type or two or more types of following measurement instruments can be freely adopted in combination: a fluorescence measurement instrument, a scattered light measurement instrument, a transmitted light measurement instrument, a reflected light measurement instrument, a diffracted light measurement instrument, an ultraviolet spectrometer, an infrared spectrometer, a Raman spectrometer, a FRET measurement instrument, a FISH measurement instrument, other various spectrum measurement instruments, a so-called multi-channel photodetector in which a plurality of photodetectors is arranged in an array, and the like.

Furthermore, in the present technology, the light detection unit 103 preferably has a light receiving element that receives light generated from the microparticle. Examples of the light receiving element can include an area imaging element such as a CCD or a CMOS element, a PMT, a photodiode, and the like.

Furthermore, the light detection unit 103 can include a plurality of light receiving elements having different detection wavelength bands. Since the light detection unit 103 includes the plurality of light receiving elements having the different detection wavelength bands, intensity of light in a continuous wavelength band can be measured as a fluorescence spectrum. Specifically, for example, it is possible to exemplify: a PMT array or photodiode array in which light receiving elements are arranged one-dimensionally; one in which a plurality of independent detection channels such as two-dimensional light receiving elements like CCDs or CMOSs is arranged; and the like.

### (5) Analysis Unit 104

The analysis unit 104 is connected to the light detection unit 103 and analyzes a detection value of light for a microparticle detected by the light detection unit 103.

The analysis unit 104 can correct, for example, a detection value of light received from the light detection unit 103 and can calculate a feature quantity of each microparticle. Specifically, the feature quantity indicating a size, a form, an internal structure, and the like of the microparticle is calculated from detection values of the received fluorescence, the forward scattered light, and the backscattered light. Additionally, a sorting control signal can also be generated by performing sorting determination on the basis of: the calculated feature quantity; a sorting condition preliminarily received from the input unit; and the like.

The analysis unit 104 is not indispensable in the particle measurement device 100 according to the present technology, and a state and the like of each microparticle can be analyzed by using an external analysis device or the like on the basis of a detection value of light detected by the light detection unit 103. For example, the analysis unit 104 may be implemented by a personal computer or a CPU, and can be made to function by the personal computer or the CPU while further storing a program in a hardware resource including recording media (nonvolatile memory (USB memory or the like), a HDD, a CD, and the like) and the like. Additionally, the analysis unit 104 may be connected to each of the units via a network.

### (6) Sorting Unit 105 (Including Electric Charging Unit 1051)

The sorting unit 105 includes at least: the vibration element 105a that generates a droplet; a deflection plate 105b that changes an electrically-charged droplet in a desired direction; and a collection container that collects droplets. The electric charging unit 1051 is separately defined in Fig. 26, but the electric charging unit is a part of the sorting unit 105 and charges electricity on the basis of a sorting control signal generated by the analysis unit 104.

In the microparticle measurement device 100 of Fig. 26, the vibration element 105a generates a droplet by applying vibration to the orifice M1 as described above. The electric charging unit 1051 charges positively or negatively the droplet discharged from the orifice M1 of the microparticle measurement chip M on the basis of a sorting control signal generated by the analysis unit 104. Then, an advancing direction of the electrically-charged droplet is changed and sorted in a desired direction by the deflection plate (counter electrode) 105b to which voltage is applied.

Note that the vibration element 105a to be used is not particularly limited and any known vibration element can be freely selected and used. As an example, a piezo vibration element or the like can be exemplified. Additionally, a size of a droplet can be adjusted by adjusting a liquid feeding amount to the flow path P, a diameter of a discharge port, a vibration frequency of the vibration element 105a, and the like, and a droplet including a constant amount of microparticles can be generated.

### (7) Memory Unit 106

The memory unit 106 stores all of matters relating to measurement, such as a value detected by the light detection unit 103, a feature quantity calculated by the analysis unit 104, a sorting control signal, and a sorting condition input from the input unit.

In the microparticle measurement device 100, the memory unit 106 is not indispensable, and an external storage device may be connected. As the memory unit 106, for example, a hard disk or the like can be used. Furthermore, the recording unit 106 may be connected to each of the units via the network.

### (8) Display Unit 107

The display unit 107 can display all of the matters relating to the measurement such as a value detected by the light detection unit 103 and a feature quantity calculated by the analysis unit 104. Preferably, the display unit 107 can display, as a scattergram, the feature quantity calculated by the analysis unit 104 for each microparticle.

In the microparticle measurement device 100, the display unit 107 is not indispensable and an external display device may be connected. As the display unit 107, for example, a display, a printer, or the like can be used.

### (9) Input Unit 108

The input unit 108 is a portion operated by a user such as an operator. A user can access the control unit through the input unit 108 to control each of the units of the microparticle measurement device 100 according to the present technology. Preferably, the input unit 108 can set an attention region for the scattergram displayed on the display unit 107 and can determine a sorting condition.

In the microparticle measurement device 100, the input unit 108 is not indispensable, and an external operating device may be connected. As the input unit 108, for example, a mouse, a keyboard, or the like can also be used.

### (10) Control Unit 109

The control unit 109 can control each of the cell sample liquid feeding device 10, the fluid control unit 101, the light emission unit 102, the light detection unit 103, the analysis unit 104, the sorting unit 105, the electric charging unit 1051, the recording unit 106, the display unit 107, and input unit 108. The control unit 109 may be provided separately for each of the units, and furthermore, may be provided outside the microparticle measurement device 100. For example, the control unit may be implemented by a personal computer or a CPU, and can be made to function by the personal computer or the CPU while further storing a program in a hardware resource including recording media (nonvolatile memory (USB memory and the like), a HDD, a CD, and the like) and the like. Additionally, the control unit 109 may be connected to each of the units via the network.

### (11) Others

The microparticle measurement device 100 according to the present technology can be housed in a biosafety cabinet. Since the microparticle measurement device is housed in the biosafety cabinet, it is possible to prevent: scattering to a surrounding region including a user; and sample contamination. However, the fluid control unit 101 is not necessarily housed in the biosafety cabinet and can be connected to the microparticle measurement device 100 at an open portion on a wall surface of the biosafety cabinet via each tube at an opened portion.

Additionally, each of the units of the microparticle measurement device 100 can be cleaned in order to prevent sample contamination. Particularly, it is preferable that a casing including the cell sample liquid feeding device 10, the flow path P, and the sorting unit 105 each of which possibly contacts the sample can be cleaned.

### REFERENCE SIGNS LIST

- 1: Cell sample liquid feeding bag
- 11: Outflow port
- 12: Bottom portion
- 121: Reservoir
- 13: First inner tube
- 14: Outer tube
- 141: Outer tube pump
- 15: Port
- 16: Second inner tube
- 17: Support portion
- 10: Cell sample liquid feeding device
- 100: Microparticle measurement device
- 101: Fluid control unit
- 1011: Sheath liquid feeding unit
- 1012: Liquid drain unit
- 102: Light emission unit
- 103: Light detection unit
- 104: Analysis unit
- 105: Sorting unit
- 105a: Vibration element
- 105b: Deflection plate
- 1051: Electric charging unit
- 106: Recording unit
- 107: Display unit
- 108: Input unit
- 109: Control unit
- P: Flow path
- T: Substrate
- M: Microparticle measurement chip
- Ma, Mb: Substrate layer
- M1: Orifice
- M11: Cut-away portion
- M2: Sample flow path
- M3: Sample introduction unit
- M4: Sheath introduction unit
- M5: Suction flow path
- M51: Suction open portion
- M52: Communication port
- M61, 62: Narrowed portion
- M7: Straight portion
- L1: Diameter of cut-away portion M11
- L2: Opening diameter of orifice M1
- C1: Liquid feeding tube
- C2: Sheath liquid feeding tube
- C3: Liquid drain tube

## Claims

1. A cell sample liquid feeding bag (1) comprising at least:
an outflow port (11) from which cell sample liquid flows out;
a bottom portion (12) at least partly including a slope; and
a first inner tube (13),
**characterized in that**
a cross-sectional shape of the bottom portion (12) has a substantially V-shape;
the bottom portion (12) includes a reservoir (121) configured to store cells; and
the first inner tube (13) extends from the outflow port (11) toward the reservoir (121) to a bottom of the V-shape, not contacting the reservoir (121), and is configured to feed cell sample liquid from a reservoir side toward an outflow port side of the first inner tube (13) and to whirl up the cells when a liquid is ejected toward the reservoir (121).

2. The cell sample liquid feeding bag (1) according to claim 1,
further comprising an outer tube (14) extending from the outflow port (11) toward outside of the bag (1),
wherein the outer tube (14) includes at least two or more branched paths; or
further comprising a support portion (17) that supports an upright posture of the bag (1).

3. The cell sample liquid feeding bag (1) according to claim 1, further comprising at least one or more ports (15) besides the outflow port (11).

4. The cell sample liquid feeding bag (1) according to claim 3, wherein the port/ports (15) is/are provided at a bag wall surface.

5. The cell sample liquid feeding bag (1) according to claim 4, wherein
one of the ports (15) further includes a second inner tube (16) extending toward an inside of the bag (1), and
the second inner tube (16) is configured for cell sample liquid injection.

6. The cell sample liquid feeding bag (1) according to claim 5, wherein an inner diameter of the second inner tube (16) is larger than an inner diameter of the first inner tube (13).

7. The cell sample liquid feeding bag (1) according to claim 1, wherein a coating that inhibits nonspecific adsorption of the sample is applied to at least a part of a bag inner wall.

8. The cell sample liquid feeding bag (1) according to claim 7, wherein the coating includes one kind selected from a group consisting of low molecular protein, silicon, and a water-soluble polymer.

9. The cell sample liquid feeding bag (1) according to claim 8,
wherein the low molecular protein includes albumin; or
wherein the water-soluble polymer includes at least one or more kinds selected from a group consisting of casein, gelatin, dextran, polyacrylamide, polyvinyl alcohol, polyvinylpyrrolidone, and polyethylene glycol.

10. A cell sample liquid feeding method using a cell sample liquid feeding bag (1) according to one of claims 1 and 3-9, the method **characterized by** at least comprising:
an ejection step of ejecting liquid from the outflow port side of the inner tube (13) toward the reservoir side before starting cell sample liquid feeding.

11. The cell sample liquid feeding method according to claim 10, wherein
the cell sample liquid feeding bag (1) further includes an outer tube (14) extending from the outflow port (11) toward outside of the bag (1), and
the ejection step is executed by reverse rotation of an outer tube pump (141).

12. A cell sample liquid feeding device (10) comprising at least a cell sample liquid feeding bag (1) according to one of claims 1 to 9.

13. The cell sample liquid feeding device (10) according to claim 12, further comprising an ejection mechanism capable of ejecting liquid from the outflow port side toward the reservoir side before feeding the cell sample liquid.

14. The cell sample liquid feeding device (10) according to claim 13, wherein
the cell sample liquid feeding bag (1) further includes an outer tube (14) extending from the outflow port (1) toward outside of the bag (1), and
the ejection mechanism is provided by reverse rotation of an outer tube pump (141).

15. A microparticle measurement device comprising at least the cell sample liquid feeding device according to one of claims 12 to 14.

## Patentansprüche

1. Beutel (1) für eine Zufuhr von Zellprobenflüssigkeit, umfassend mindestens:
einen Ausflussanschluss (11), aus der die Zellprobenflüssigkeit herausfließt;
einen Bodenabschnitt (12), der mindestens teilweise eine Neigung einschließt; und
einen ersten Innenschlauch (13),
**dadurch gekennzeichnet, dass**
eine Querschnittsform des Bodenabschnitts (12) im Wesentlichen eine V-Form aufweist;
der Bodenabschnitt (12) ein Reservoir (121) einschließt, das konfiguriert ist, um Zellen aufzubewahren; und
der erste Innenschlauch (13) sich von dem Ausflussanschluss (11) zu dem Reservoir (121) hin bis zu einem Boden der V-Form erstreckt, ohne das Reservoir (121) zu berühren, und konfiguriert ist, um Zellprobenflüssigkeit von einer Reservoirseite zu einer Ausflussanschlussseite des ersten Innenschlauchs (13) zuzuführen und um die Zellen aufzuwirbeln, wenn eine Flüssigkeit zu dem Reservoir (121) hin ausgestoßen wird.

2. Beutel (1) für eine Zufuhr von Zellprobenflüssigkeit nach Anspruch 1,
ferner umfassend einen Außenschlauch (14), der sich von dem Ausflussanschluss (11) zu einer Außenseite des Beutels (1) hin erstreckt,
wobei der Außenschlauch (14) mindestens zwei oder mehr verzweigte Pfade einschließt; oder
ferner umfassend einen Stützabschnitt (17), der eine aufrechte Haltung des Beutels (1) unterstützt.

3. Beutel (1) für eine Zufuhr von Zellprobenflüssigkeit nach Anspruch 1, ferner umfassend neben dem Ausflussanschluss (11) mindestens einen oder mehrere Anschlüsse (15).

4. Beutel (1) für eine Zufuhr von Zellprobenflüssigkeit nach Anspruch 3, wobei der Anschluss/die Anschlüsse (15) an einer Beutelwandoberfläche bereitgestellt ist/sind.

5. Beutel (1) für eine Zufuhr von Zellprobenflüssigkeit nach Anspruch 4, wobei
einer der Anschlüsse (15) ferner einen zweiten Innenschlauch (16) einschließt, der sich zu einer Innenseite des Beutels (1) hin erstreckt, und
der zweite Innenschlauch (16) für die Injektion von Zellprobenflüssigkeit konfiguriert ist.

6. Beutel (1) für eine Zufuhr von Zellprobenflüssigkeit nach Anspruch 5, wobei ein Innendurchmesser des zweiten Innenschlauchs (16) größer ist als ein Innendurchmesser des ersten Innenschlauchs (13).

7. Beutel (1) für eine Zufuhr von Zellprobenflüssigkeit nach Anspruch 1, wobei auf mindestens einen Teil einer Innenwand des Beutels eine Beschichtung aufgebracht ist, die eine unspezifische Adsorption der Probe verhindert.

8. Beutel (1) für eine Zufuhr von Zellprobenflüssigkeit nach Anspruch 7, wobei die Beschichtung eine Art einschließt, die aus einer Gruppe ausgewählt ist, bestehend aus niedermolekularem Protein, Silikon und einem wasserlöslichen Polymer.

9. Beutel (1) für eine Zufuhr von Zellprobenflüssigkeit nach Anspruch 8,
wobei das niedermolekulare Protein Albumin einschließt,
oder
wobei das wasserlösliche Polymer mindestens eine oder mehrere Arten einschließt, die aus der Gruppe ausgewählt sind, bestehend aus Casein, Gelatine, Dextran, Polyacrylamid, Polyvinylalkohol, Polyvinylpyrrolidon und Polyethylenglykol.

10. Verfahren für eine Zufuhr von Zellprobenflüssigkeit unter Verwendung eines Beutels (1) für eine Zufuhr von Zellprobenflüssigkeit nach einem der Ansprüche 1 und 3 bis 9, wobei das Verfahren
**dadurch gekennzeichnet ist, dass** es mindestens umfasst:
einen Ausstoßschritt des Ausstoßens von Flüssigkeit von der Ausflussanschlussseite des Innenschlauchs (13) zu der Reservoirseite hin, bevor mit der Zufuhr der Zellprobenflüssigkeit begonnen wird.

11. Verfahren für eine Zufuhr von Zellprobenflüssigkeit nach Anspruch 10, wobei
der Beutel (1) für eine Zufuhr von Zellprobenflüssigkeit ferner einen Außenschlauch (14) einschließt, der sich von dem Ausflussanschluss (11) zu der Außenseite des Beutels (1) hin erstreckt, und
der Ausstoßschritt durch Rückwärtsdrehung einer Außenschlauchpumpe (141) ausgeführt wird.

12. Vorrichtung (10) für eine Zufuhr von Zellprobenflüssigkeit, umfassend mindestens einen Beutel (1) für eine Zufuhr von Zellprobenflüssigkeit nach einem der Ansprüche 1 bis 9.

13. Vorrichtung (10) für eine Zufuhr von Zellprobenflüssigkeit nach Anspruch 12, ferner umfassend einen Ausstoßmechanismus, der in der Lage ist, Flüssigkeit von der Ausflussanschlussseite zu der Reservoirseite hin auszustoßen, bevor die Zellprobenflüssigkeit zugeführt wird.

14. Vorrichtung (10) für eine Zufuhr von Zellprobenflüssigkeit nach Anspruch 13, wobei
der Beutel (1) für eine Zufuhr von Zellprobenflüssigkeit ferner einen Außenschlauch (14) einschließt, der sich von dem Ausflussanschluss (1) zu der Außenseite des Beutels (1) hin erstreckt, und
der Ausstoßmechanismus durch Rückwärtsdrehung einer Außenschlauchpumpe (141) bereitgestellt wird.

15. Mikropartikelmessvorrichtung, umfassend mindestens die Vorrichtung für eine Zufuhr von Zellprobenflüssigkeit nach einem der Ansprüche 12 bis 14.

## Revendications

1. Poche d'alimentation en liquide d'échantillon cellulaire (1) comprenant au moins :
un orifice d'écoulement de sortie (11) à partir duquel un liquide d'échantillon cellulaire s'écoule vers l'extérieur ;
une partie inférieure (12) comportant au moins partiellement une pente ; et
un premier tube interne (13),
**caractérisée en ce que**
une forme en coupe transversale de la partie inférieure (12) a sensiblement une forme de V ;
la partie inférieure (12) comporte un réservoir (121) conçu pour stocker des cellules ; et
le premier tube interne (13) s'étend à partir de l'orifice d'écoulement de sortie (11) en direction du réservoir (121) jusqu'au-dessous de la forme de V, ne venant pas en contact avec le réservoir (121), et est conçu pour alimenter un liquide d'échantillon cellulaire à partir d'un côté réservoir en direction d'un côté orifice d'écoulement de sortie du premier tube interne (13) et pour faire tourbillonner les cellules lorsqu'un liquide est éjecté en direction du réservoir (121).

2. Poche d'alimentation en liquide d'échantillon cellulaire (1) selon la revendication 1,
comprenant en outre un tube externe (14) s'étendant à partir de l'orifice d'écoulement de sortie (11) vers l'extérieur de la poche (1),
dans laquelle le tube externe (14) comporte au moins deux trajets ramifiés ou plus ; ou
comprenant en outre une portion de support (17) qui supporte une posture dressée de la poche (1).

3. Poche d'alimentation en liquide d'échantillon cellulaire (1) selon la revendication 1, comprenant en outre au moins un ou plusieurs orifices (15) en plus de l'orifice d'écoulement de sortie (11).

4. Poche d'alimentation en liquide d'échantillon cellulaire (1) selon la revendication 3, dans laquelle l'orifice/les orifices (15) est/sont fourni(s) au niveau d'une surface de paroi de poche.

5. Poche d'alimentation en liquide d'échantillon cellulaire (1) selon la revendication 4, dans laquelle
l'un des orifices (15) comporte en outre un second tube interne (16) s'étendant vers l'intérieur de la poche (1), et
le second tube interne (16) est conçu pour une injection de liquide d'échantillon cellulaire.

6. Poche d'alimentation en liquide d'échantillon cellulaire (1) selon la revendication 5, dans laquelle un diamètre interne du second tube interne (16) est plus grand qu'un diamètre interne du premier tube interne (13).

7. Poche d'alimentation en liquide d'échantillon cellulaire (1) selon la revendication 1, dans laquelle un revêtement qui inhibe une adsorption non spécifique de l'échantillon est appliqué à au moins une partie d'une paroi interne de poche.

8. Poche d'alimentation en liquide d'échantillon cellulaire (1) selon la revendication 7, dans laquelle le revêtement comporte un type choisi dans un groupe constitué par protéine à faible masse moléculaire, silicium et un polymère hydrosoluble.

9. Poche d'alimentation en liquide d'échantillon cellulaire (1) selon la revendication 8,
dans laquelle la protéine à faible masse moléculaire comporte de l'albumine ;
ou
dans laquelle le polymère hydrosoluble comporte au moins un ou plusieurs types choisis dans un groupe constitué par caséine, gélatine, dextrane, polyacrylamide, alcool polyvinylique, polyvinylpyrrolidone et polyéthylène glycol.

10. Procédé d'alimentation en liquide d'échantillon cellulaire à l'aide d'une poche d'alimentation en liquide d'échantillon cellulaire (1) selon l'une des revendications 1 et 3 à 9, le procédé **caractérisé en ce qu'**il comprend au moins :
une étape d'éjection consistant à éjecter un liquide à partir d'un côté orifice d'écoulement de sortie du tube interne (13) en direction du côté réservoir avant le début d'une alimentation en liquide d'échantillon cellulaire.

11. Procédé d'alimentation en liquide d'échantillon cellulaire selon la revendication 10, dans lequel
la poche d'alimentation en liquide d'échantillon cellulaire (1) comporte en outre un tube externe (14) s'étendant à partir de l'orifice d'écoulement de sortie (11) vers l'extérieur de la poche (1), et
l'étape d'éjection est exécutée par rotation inverse d'une pompe de tube externe (141).

12. Dispositif d'alimentation en liquide d'échantillon cellulaire (10) comprenant au moins une poche d'alimentation en liquide d'échantillon cellulaire (1) selon l'une des revendications 1 à 9.

13. Dispositif d'alimentation en liquide d'échantillon cellulaire (10) selon la revendication 12, comprenant en outre un mécanisme d'éjection capable d'éjecter un liquide à partir du côté orifice d'écoulement de sortie en direction du côté réservoir avant alimentation du liquide d'échantillon cellulaire.

14. Dispositif d'alimentation en liquide d'échantillon cellulaire (10) selon la revendication 13, dans lequel
la poche d'alimentation en liquide d'échantillon cellulaire (1) comporte en outre un tube externe (14) s'étendant à partir de l'orifice d'écoulement de sortie (1) vers l'extérieur de la poche (1), et
le mécanisme d'éjection est fourni par rotation inverse d'une pompe de tube externe (141).

15. Dispositif de mesure de microparticules comprenant au moins le dispositif d'alimentation en liquide d'échantillon cellulaire selon l'une des revendications 12 à 14.
